Europäisches Patentamt

(19) European Patent Office

Office européen des brevets

(11) Publication number: **0 227 259**
**A1**

# (12) EUROPEAN PATENT APPLICATION

(21) Application number: **86308351.5**

(22) Date of filing: **27.10.86**

(51) Int. Cl.⁴: **C 07 C 7/10**
**C 07 C 7/11, B 01 D 53/14**
**C 10 G 21/20**

(30) Priority: **28.10.85 US 791661**

(43) Date of publication of application:
**01.07.87 Bulletin 87/27**

(84) Designated Contracting States:
**GB NL SE**

(71) Applicant: **THE DOW CHEMICAL COMPANY**
**2030 Dow Center Abbott Road P.O. Box 1967**
**Midland, MI 48640(US)**

(72) Inventor: **Pearce, Roscoe L.**
**313 Narcissus**
**Lake Jackson Texas 77566(US)**

(72) Inventor: **Wolcott, Richard A.**
**103 Finch Lane**
**Angleton Texas 77515(US)**

(74) Representative: **Burford, Anthony Frederick et al,**
**W.H. Beck, Greener & Co. 7 Stone Buildings Lincoln's Inn**
**London WC2A 3SZ(GB)**

(54) **Sulfur removal from hydrocarbons.**

(57) Liquid and gaseous hydrocarbon streams are treated to remove COS by contacting the hydrocarbon streams with an aqueous solution of selective $H_2S$-absorbent and a COS absorbent/hydrolyzer whereby COS is hydrolyzed to $H_2S$ and $CO_2$ and said products are absorbed by the $H_2S$ absorbent in the selective range thereof. Preferably, the $H_2S$ absorbent is an alkanolamine, especially methyldiethanolamine, and the COS absorbent/hydrolyzer is an organic liquid, especially diisopropanolamine. The resultant hydrocarbon stream can be further treated in one or more subsequent steps with aqueous caustic solution containing, in some steps, a primary alkanolamine, especially monoethanolamine, to remove acid gases and, in particular, to reduce the COS content to below 1,000 ppb.

EP 0 227 259 A1

## SULFUR REMOVAL FROM HYDROCARBONS

Natural gases and liquid hydrocarbons are known to contain acid gases such as carbon dioxide and one or more sulfur containing components such as carbonyl sulfide (COS), hydrogen sulfide ($H_2S$), mercaptans and the like, many of which must be removed to make the hydrocarbons suitable for many uses, such as polymerizations, combustion, and the like, because of the deleterious effect these gases have on various factors in the products and environment.

The term "liquid" as used throughout the specification (including the claims) includes liquified hydrocarbons, especially liquified petroleum gases.

Carbonyl sulfide (COS) is contained in natural gas in small quantities ie. 50 to 500 ppm (parts per million) and in liquid hydrocarbon streams in concentrations in the 1 to 100 ppm range. The specification level in treated products is in the 1 ppm range or less for natural gas and most liquid streams. There are several processes available for removing COS to these levels.

However, some specifications for liquid hydrocarbon products are in the 50 to 1000 ppb (parts per billion) range. For example, ethylene and propylene for polyethylene and polypropylene manufacture respectively, normally have a 50 or less ppb limit. There are no known techniques for removing COS to these low levels, e.g. ppb, which are commercially viable. The removal of carbonyl sulfide (COS) is very poor in caustic solutions. No more than 10 to 15 percent removal can be expected in conventional caustic solutions used in the conventional designed contactor.

There are, of course, several processes which employ physical solvents and/or solvents which aid in the hydrolysis of COS to its component $CO_2$ and $H_2S$, but most of these processes have high make-up rates because the solvents and/or co-solvents are soluble in the liquid hydrocarbons to varying degrees requiring excessive make-up and thus are uneconomical in commercial processes.

In addition most of the materials for removing the COS to the ppm level usually form products which are either impure and command a very poor price or are objectionable from the environmental standpoint.

While mercaptans (RSH) are contained in some natural gases, essentially all refinery liquid hydrocarbon streams contain mercaptans. The specifications for refinery use and for polymerization reactions employing these products usually require the mercaptan level to be in the 1 to 20 ppm range.

The known art for the removal of mercaptans consists of one to three stages of contact with an aqueous caustic solution containing some type of solubilizer. A separate unit is required for these operations. An example of one such process is the Merox Process requiring a separate unit in which the mercaptans are converted to their disulfides. The Merox solution is a caustic solution which contains a proprietary catalyst which is necessary for the economic conversion of RSH to RSSR. The art as practiced normally uses formulated caustic solution (ie. containing catalyst or small quantity of cosolvent) to improve the solubility of the RSH and/or to increase the rate of reaction in the caustic solution because RSH removal is poor in caustic alone, particularly with respect to removal of the higher molecular weight mercaptans, eg. butyl and higher alkyl mercaptans.

Some amines that have been considered for the sulfur or mercaptan removal are discussed below.

The advantages and disadvantages of using monoethanolamine (MEA) in a refinery include some of the following:

Advantages
1.  It has the capability of producing the lowest level of $H_2S$ and $CO_2$ in the product but has little or no selectivity required for tail gas sulfur producing processes.
2.  It can be partially reclaimed in the event of thermal degradation or build-up of heat stable salts but often the high MEA usage rates result from these reclaiming operations (necessitated by the irreversible reaction of COS and MEA) as well as losses to the products because of MEA's solubility in the products.

3. It can hydrolyze COS and thus the product meets fairly low COS specifications.

## Disadvantages

1. It has high heats of reaction with $H_2S$ and $CO_2$.
2. It lacks selectivity and in applications where this is a preference, energy requirements are further increased.
3. It has a higher solubility in liquid hydrocarbon streams than many other amines.
4. Corrosion potential limits solution strength to about 15 percent by weight.
5. A portion of the COS removed reacts irreversibly with the MEA causing losses.

When diethanolamine (DEA) is considered as a replacement for MEA, its advantages and disadvantages include some of the following:

## Advantages

1. It has lower heats of reaction.
2. It has a slight selectivity for $H_2S$ over $CO_2$.
3. It is slightly less soluble in liquid hydrocarbons.
4. It can remove COS in some cases to acceptable levels.

## Disadvantages

1. It has insufficient selectivity for tail gas treating.
2. Reclaiming is not a common, straight-forward process.
3. It forms irreversible products with $CO_2$ creating losses of the absorbent.

34,380-F                    -4-

4.   It doesn't produce treated gas specifications as low as MEA.

A refinery choosing methyldiethanolamine (MDEA) as a replacement for MEA and/or DEA does so based on the following advantages out-weighing the disadvantages:

Advantages

1.   It has still lower heats of reaction than either MEA or DEA.

2.   It has the required $H_2S$ to $CO_2$ selectivity required for tail gas treating and other gas streams containing $CO_2$ and $H_2S$.

3.   It is slightly less soluble in liquid hydrocarbons.

4.   It is more resistant to chemical degradation.

5.   It is not corrosive.

6.   Solution strengths up to 50 percent can be used for added acid gas removal capacity.

Disadvantages

1.   It doesn't produce treated gas specifications as low as MEA or DEA.

2.   It isn't known for its ability to remove COS.

3.   Reclaiming is feasible but somewhat more difficult than MEA but not as complex as DEA.

4.   Solvent cost is higher.

It would be advantageous to have a plant designed to remove all of these acid gases to the aforesaid levels producing a hydrocarbon product useful in the many latter processes.  Such a designed acid gas

34,380-F                    -5-

treating plant [liquid and gas are indicated by the trade in the term "gas treating plant"] is described consisting of a series of unit operations which integrate into the existing processes both from the mode of operation and the equipment which is used to produce commercially soluble products for polymerizations; combustions and the like.

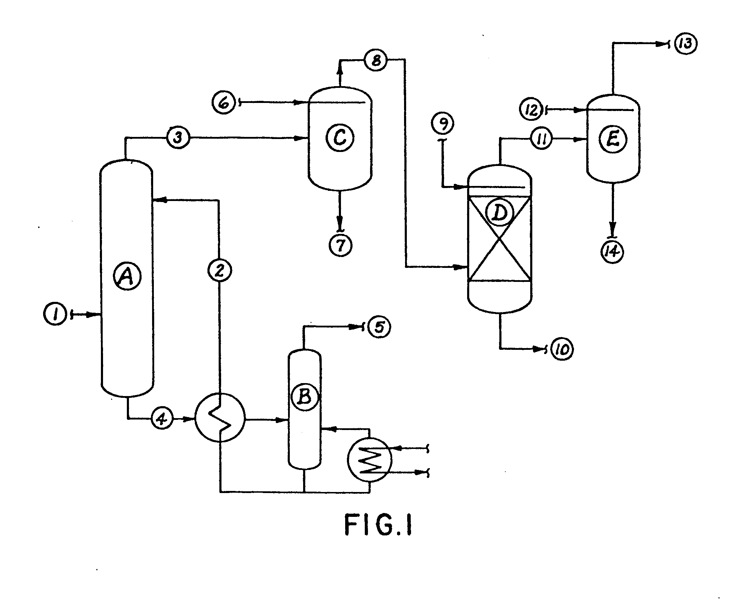

In the accompanying Figure 1, a flow diagram of a light hydrocarbon acid gas removal plant is shown in accordance with the present invention in which the acid gas content can be reduced to below about 100 ppb.

Throughout the application all percentages (%) are by weight unless otherwise indicated. Also same of the more commonly used abbreviations herein mean:

MEA = monoethanolamine
DEA = diethanolamine
MDEA = methyldiethanolamine
DIPA = diisopropanolamine

In accordance with the present invention a natural or synthetic gas stream or liquid hydrocarbon stream as for example, a $C_3/C_4$ stream from a refinery debutanizer which contains acid gases such as $H_2S$, $CO_2$, COS, mercaptans (methyl, ethyl, propyl, butyl and even higher alkyl moieties) can be freed of these undesirable components by:

1) Treatment of the gas or liquid stream with a formulated aqueous absorbent containing a selective $H_2S$ absorbent, e.g. MDEA and a highly active COS absorbent/hydrolyzer, e.g.

DIPA wherein the $H_2S$ is selectively absorbed and COS hydrolyzed to $H_2S$ and $CO_2$, the products being absorbed by the selective $H_2S$ absorbent in the desired selective range. The formulated absorbent is regenerated (stripped) and returned to the absorber. Some make up of COS absorbent/ hydrolyzer is necessary when liquid hydrocarbons are treated because of solubility of these classes of compounds in the liquid hydrocarbons.

Thus, the present process concerns treating liquid and gaseous hydrocarbon streams containing acid gases, including $H_2S$, $CO_2$, COS and mercaptans, to remove a substantial portion of said acid gases which comprises:

contacting said hydrocarbon stream containing $H_2S$, $CO_2$, COS, and mercaptans with an alkanolamine which is a selective absorbent for $H_2S$ with respect to $CO_2$ which solution also contains an organic liquid COS absorbent.

The so-treated gas stream or liquid hydrocarbon will be found to have from 30 to 80 percent of the COS removed as compared with only from 10 to 30 percent when a selective amine is used alone.

Further, by employing a selective $H_2S$ absorbent at this stage of the process the $H_2S$ to $CO_2$ ratio picked up by the absorbent can be adjusted to provide a regenerator gas product suitable for economical operations of most commercial sulfur recovery processes, e.g. a Claus Unit. In addition, since the COS is partially converted to $H_2S$ and $CO_2$, later treatments to

34,380-F

remove the other acid gases are able to produce less contaminated waste streams, many of which can be after-treated to useful products and the treating solutions regenerated to provide more economical operations. Substantially any of the secondary and/or tertiary alkanolamines can be employed in this step, each having a recognized advantage under the operating parameters of absorber operations, feed stream conditions and available battery utilities. The exemplary discussions employ MDEA which, based on the ratio of $H_2S$ and $CO_2$ in the feed stream, provided a regenerator off-gas with an $H_2S$ to $CO_2$ ratio to fit an existing Claus Unit operating parameters in the refinery. Other sulfur conversion processes as well as a similar process on a different scale may require other ratios of $H_2S$ to $CO_2$ which will dictate the selection of the selective absorbent. The selectivity of the various absorbents, particularily the amines and more particularily the secondary and tertiary alkanolamines are well documented in the literature and one can be found without undue experimentation to give the most effective use of existing equipment.

In addition, by using a selective $H_2S$ absorbent formulated to contain an organic liquid COS solvent (absorbent) the losses of both solvent absorbents is reduced since most selective $H_2S$ sorbents are less soluble in liquid hydrocarbon streams and smaller quantities of the COS absorbent are possible thus reducing its losses.

2) Treatment of the resulting product gas or liquid stream with an aqueous formulated alkali (caustic) solution wherein the contact time is such to remove from 50 to 80 percent of the remaining COS but "slip" most of the mercaptans. Usually, the concentration of caustic is in the range of 5 to 50 percent by weight and the alkanolamine, preferably a primary alkanolamine, in from about 0.5 to 20 percent by weight. It has been found advantageous to employ a single stage contactor. The COS being converted herein to $Na_2S$ and $Na_2CO_3$ may be used to generate elemental sulfur and $CO_2$ or used per se in several industrial processes, e.g. paper production.

3) The gas or liquid stream usually is then contacted with a 5 to 50 and preferably a 10 to 25 percent by weight of an unformulated aqueous alkali solution (NaOH) in a "structured" packed zone of several stages, preferably a minimum of six stages. Here essentially all of the mercaptans are removed as well as an additional 70 to 90 plus percent of the remaining COS. The waste stream from this treatment may be regenerated in known ways.

4) Following these treatments the stream, if liquid, usually is washed with water to remove residual alkali, and again, if a liquid stream is from for example a debutanizer, it is split into its components, e.g. $C_3$ and $C_4$ and the $C_3$ or the gas, if a natural or synthetic source, is "polished" substantially free of COS, which of course goes with the lower boiling components ($C_3$) or remains in the gas streams, by contacting it with a final formulated aqueous alkali/alkanolamine solution in a structured multi-stage contactor.

The product is finally washed to remove any traces of alkali or alkanolamine and .is found to be substantially free of sulfur containing organic and inorganic acid gases with COS in the range of less than 1000 ppb and usually less than about 100 ppb.

It is of course to be understood that any of the above steps may be eliminated, by-passed or included, with greater or reduced contact times, recirculation rates and/or strengths if more sulfur removal is required or less sulfur removal is acceptable, respectively.

The formulated solutions employed in accordance with the present invention are of two general types:

a)    a selective $H_2S$ absorbent such as MDEA, and a COS absorbent/hydrolyzer under the conditions of selective $H_2S$ absorption such as DIPA; and,

b)    alkali metal hydroxides containing various amounts of at least one alkanolamine.

In addition it is advantageous to employ "structured" packing having a high surface-to-volume ratio, such as Goodloe knitted packing, in the formulated alkali/alkanolamine treatment steps.

The percent removal of each component is related to the strength of the treating solutions, the contact time, and the loadings are dependent on the circulation rates.

34,380-F                              -10-

The first step treatment, the combined primary $H_2S$ removal step and COS initial hydrolysis step, usually is carried out in a multi-stage contactor or absorber designed to provide a contact of the gas or liquid hydrocarbons with the formulated $H_2S$/COS absorbent/hydrolysis solution of from 50 to 120 seconds. Such contact times give enhanced COS removal, maximize the selective removal of $H_2S$ vis-a-vis $CO_2$, minimize solubility of the COS hydrolyzer in liquid hydrocarbon and yet permit $H_2S$ loading of about 0.2 moles $H_2S$ per mole of selective $H_2S$ absorbent.

Temperature of the selective absorbent/COS hydrolysis contact should be in the range of 40 to 90°C.

The temperature of the alkali treatments, both formulated and unformulated, ranges from 40 to 70°C.

The usual concentration of the components of the various formulations is as follows:

(a) formulated $H_2S$/COS hydrolysis solutions of from 5 to 60 percent by weight are operable but, preferably these solutions contain 20 to 50 percent by weight of the $H_2S$ absorbent and from 0.5 to 15 percent by weight and preferably from 1 to 10 percent of the COS absorbent hydrolyzer;

(b) formulated alkali/alkanolamine solutions contain 5 to 50 percent and preferably contain 5 to 25 percent of the alkali metal hydroxide and 0.5 to 20 percent and preferably 2 to 15 percent of the primary alkanolamine;

(c) the unformulated alkali scrubber contains 5 to 50 percent by weight and preferably 10 to 25 percent by weight alkali metal hydroxide solutions.

The loading of the $H_2S$ absorber/COS hydrolyzer solution is generally held below about 0.25 moles $H_2S$ per mole absorbent and preferably at about 0.2 moles.

The scope of selective $H_2S$ absorbents operable in accordance with the present inention is as wide as the known art but for energy conservation, as selective absorbent, a tertiary amine such as MDEA or diethylethanolamine is preferred, since total heat duty is about half that of, for example, MEA at 20 percent concentrations. Other selective alkanolamine absorbents are well documented relative to heat duty and can be selected on the basis of each installation design, available heating and cooling sources outside the battery of the present invention, and selectively of pick-up.

In a representative example with specific reference to Figure 1, which is a schematic of the portion of a light hydrocarbon processing plant of a refinery, a natural or synthetic gas stream or a liquid or liquified petroleum hydrocarbon stream (1) was fed to a multi-stage absorber (A) of conventional design to employ an alkanolamine to remove acid gases. In the representative example the original absorbent was monoethanolamine (MEA). The absorbent stream (2) in accordance with the present invention was a lean formulated selective $H_2S$ alkanolamine absorbent [methyldiethanolamine (MDEA) containing diisopropanolamine (DIPA)] which replaced the MEA absorbent previously used. The rich sorbent (4) was withdrawn from the bottom of the absorber and contained the absorbates, $H_2S$ and $CO_2$ tied-up in the absorbent. A major portion

34,380-F                    -12-

of the COS had been hydrolyzed to $H_2S$ and $CO_2$ which were of course picked-up by the sorbents in their selective ratios. The rich absorbent (4) was regenerated in a stripper (B), the resulting hot lean sorbent cooled by cross exchange with the cold rich stream (4), and the lean stream (2) returned to the absorber (A). The off-gas from the stripper, stream (5) contained $H_2S$ and $CO_2$ in a ratio which was suitable for sulfur recovery in, for example, a Claus sulfur unit. The hydrocarbon stream exiting the absorber, stream (3) contained very little $H_2S$, the "slipped" $CO_2$, the mercaptans and still contained from 70 to 20 percent of the COS that had been in the incoming hydrocarbon stream (1).

The treated hydrocarbon is sent to a single stage contactor (C) where it is contacted with formulated caustic solution aqueous sodium hydroxide containing monoethanolamine (MEA) stream (6). The flow rates are adjusted to provide a pick up of above 50 to 80 percent of the remaining COS and most of the $CO_2$ (COS + NaOH → NaS + $Na_2CO_3$) and "slip" a major portion of the mercaptans. The hydrocarbon stream (8) now contains only traces (ppm) of $H_2S$, and $CO_2$, the major portion of the mercaptans and some residual COS.

This stream, stream (8) was treated with an aqueous caustic solution over a structured packing, e.g. Goodloe woven mesh in column (D). Here, the intimate multi-stage contact of the stream with the caustic permits the mercaptans to be converted to their salt form and pass downwardly with the aqueous caustic treating solution while the hydrocarbons pass upwardly and out of the treater.

0227259

The hydrocarbon gas overhead (11) from column (D) is washed in column (E) with water (12). The waste wash water (14) contains traces of caustic and MEA. The treated hydrocarbon gas (13) is now free of substantially all acid gases, less than 100 ppb, and any residual of treating solutions.

At this point after column (D) the major sulfur containing compounds (acid gases) have been removed and in many cases the residual of these gases remaining is insignificant and the hydrocarbons can be used directly in downstream processes. However, should lower level acid gas contents be required, the hydrocarbon stream can be polished to remove the acid gases,particularily the COS,to parts per billion by a subsequent treatment with a formulated aqueous caustic solution in a contactor packed with a structured packing. For example in the liquid hydrocarbon section of the refinery,it is customary to fractionate the treated streams of mixed hydrocarbons and,if such is the design,it will be obvious that the COS and most other acid gases will go with the lighter fractions of the hydrocarbons. Such treatment will increase the concentration of the acid gases in the light end, in the case of the refinery treating a debutanizer stream, or mixture of $C_3$'s and $C_4$'s, the concentration of COS will about double in the $C_3$ overhead, and if this stream is to be used in the polymerization reactions, must be again treated to remove the COS to less than 50 ppb. By following the steps as herein set forth, COS and total sulfur in hydrocarbon streams can be reduced to less than 50 ppb.

Table I following illustrates the compounds present at each stage in Figure 1 as discussed above.

34,380-F

## TABLE I

### FIGURE 1 REFERENCE

| COMPOUNDS | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| Hydrocarbon | X | | X | | | | | X | | | X | | X | |
| MEA[1] | | | | | | X | | | | | | | | t[4] |
| MDEA[2] | | X | | X | | | | | | | | | | |
| DIPA[3] | | X | | X | | | | | | | | | | |
| NaOH | | | | | | X | | | X | | | | | t |
| $H_2O$ | | X | | X | | X | X | | X | X | | X | | X |
| COS | X | | Q[5] | | | | | t | | | a[6] | | a | |
| $H_2S$ | X | | t | s[7] | X | | | | | | a | | a | |
| $CO_2$ | X | | X | s | X | | | X | | | a | | a | |
| Mercaptan | X | | X | | | | | X | | | g[8] | | a | |
| $Na_2S$ | | | | | | | X | | | t | | | | t |
| $Na_2CO_3/NaHCO_3$ | | | | | | | X | | | X | | | | t |

[1] = monoethanolamine
[2] = methyldiethanolamine
[3] = diisopropanolamine
[4] = trace amount
[5] = 1/2 to 1/8 of the COS entering is found in the stream (3)
[6] = less than 100 ppb of each a and g were found in (11) and (13)
[7] = the $H_2S$ or $CO_2$ salt of an amine is formed
[8] = RSNa formed

0227259

The following data In Table II illustrates the significant improvement in acid gas removal from the light hydrocarbons processing streams of one refinery.

A refinery debutanizer product was employed which consists mainly of $C_3$ and $C_4$ liquified petroleum gases containing $H_2S$, COS and mercaptans ($C_{1-4+}$). This section of the plant was operated using MEA, then MDEA and finally formulated MDEA with the formulated alkali in accordance with the present invention. The following Table II documents the results using the various formulated solutions of the present invention compared to the original solutions starting with MEA, changing MEA to MDEA then the formulated MDEA solutions; Example 4 is projected from experience based on Example 3 using higher concentrations of MDEA and 2 percent DIPA.

## TABLE II

| Run Number | 1 | 2 | 3 | 4 |
|---|---|---|---|---|
| Solvent | MEA | MDEA | MDEA[1] | MDEA[1] |
| Conc. wt/% | 15 (67) | 20 (90) | 20 (90) | 50 (225) |
| Circ. rate gpm[5] (1pm) | 1179 | 1179 | 1179 | 462 |
| X-Exch.[4] temp °F (°C) | 30 (−1) | 30 (−1) | 30 (−1) | 30 (−1) |
| Reflux $H_2O$/acid gas | 1.2 | 1.2 | 1.2 | 1.2 |
| Heat of Reaction | | | | |
|     Calc. MMBTUH[6] (GJ) | 7419 (7827) | 4304 (4541) | 4304 (4541) | 4304 (4541) |
| Reflux :Latent Heat | | | | |
|     MMBTUH (GJ) | 6218 (6566) | 5561 (5867) | 5561 (5867) | 5561 (5867) |
| Sensible Heat | | | | |
|     MMBTUH (GJ) | 17292 (18243) | 16989 (17923) | 16989 (17923) | 6516 (6874) |
| Reboiler duty | | | | |
|     MMBTUH (GJ) | 30929 (32630) | 51204 (54020) | 26854 (28331) | 16381 (17282) |
| COS before contactor (ppm) | | 3 | 3 | 3 |
|     after contactor (ppm) | | 2 | 1 | 1' |
|     after caustic scrubber (ppm) | | 2[2] | 0.05–0.1[3] | 0.05–0.1[3] |

[1] formulated with about 2% DIPA
[2] 15% NaOH
[3] 15% formulated NaOH
[4] X-Exch. is heat exchanger
[5] gallons per minute = 4.5 litre per minute (1pm)
[6] MMBTUH means million BTU per hour =
    1.055 gigajoules (GJ)

In another example a synthetic gas stream containing 3.5 volume percent $H_2S$, 500 ppm COS, 2500 ppm each of methyl and ethyl mercaptan was feed at 50°C and 50 psig (350 KPa) for COS removal through a high surface area to volume structured packing (Goodloe unit packing) at various liquid to gas ratios (L/G) and temperatures to obtain the effect of L/G and temperature on COS removal in the presence of RSH (mercaptans) and $H_2S$ by a 10 percent sodium hydroxide, 90 percent water solution. The results are listed below in Table III as well as a single stage contactor results which illustrates the benefit of the inclusion of such a unit operation in the preferred embodiment of the present invention.

Table III

Caustic Removal of H$_2$S, COS, EtSH, and MeSH

| Solution | L/G | Temp.°C | % Removal of Acid Gases | | | |
| --- | --- | --- | --- | --- | --- | --- |
| | | | COS | H$_2$S | EtSH | MeSH |
| 10% NaOH and 90% Water Solution | .012 | 50 | 70 | 99.9 | 99.0 | 99.0 |
| | .012 | 55 | 80 | 99.9 | 99.2 | 99.2 |
| | .012 | 60 | 92 | 99.9 | 99.4 | 99.4 |
| | .016 | 50 | 70 | 99.9 | 99.0 | 99.4 |
| | .03 | 50 | -- | 99.9 | 99.5 | 99.5 |
| | .03 | 50 | -- | 99.9 | 99.5 | 99.5 |
| | .034 | 50 | -- | 99.9 | 99.6 | 99.6 |
| | .034 | 50 | -- | 99.9 | 99.9 | 99.9 |
| | .035 | 50 | -- | 99.9 | 99.6 | 99.6 |
| | .041 | 50 | 68 | 99.9 | 99.6 | 99.7 |
| | .043 | 94 | 90 | 99.9 | 99.6 | 99.8 |
| | .046 | 50 | 80 | 99.9 | 99.9 | 99.9 |
| | .047 | 50 | 80 | 99.9 | 99.8 | 99.9 |
| | .047 | 85 | 94 | 99.9 | 99.7 | 99.8 |
| | .053 | 50 | 90 | 99.9 | 99.6 | 99.6 |
| | .053 | 50 | 66 | 99.9 | 99.6 | 99.6 |
| | .058 | 85 | 78 | 99.9 | 99.7 | 99.8 |
| | .064 | 50 | 82 | 99.9 | 99.8 | 99.9 |
| | .064 | 85 | 94 | 99.9 | 99.7 | 99.8 |
| | .069 | 50 | 82 | 99.9 | 99.9 | 99.9 |
| | .079 | 50 | 80 | 99.9 | 99.7 | 99.8 |
| No packing in column same solution | .012 | 50 | 0 | 95.7 | 89.2 | 91.9 |

0 indicates 0% removed
-- indicates that the component was present but was not analyzed.

CLAIMS

1. A process for treating liquid and gaseous hydrocarbon streams containing acid gases, including $H_2S$, $CO_2$, COS and, when present, mercaptans to remove a substantial portion of said acid gases which comprises:

contacting said hydrocarbon stream with an aqueous composition containing an alkanolamine which is a selective absorbent for $H_2S$ with respect to $CO_2$ and a COS absorbent/hydrolyzer whereby COS is hydrolyzed to $H_2S$ and $CO_2$ and said products are absorbed by the $H_2S$ absorbent in the selective range thereof.

2. A process as claimed in Claim 1, wherein the COS absorbent/hydrolyzer is an organic liquid.

3. A process as claimed in Claim 2, wherein the alkanolamine is methyldiethanolamine and the COS absorbent/hydrolyzer is diisopropanolamine.

4. A process as claimed in any one of the preceding claim, wherein the $H_2S$ absorbent is employed in from 5 to 60 percent and the COS absorbent/hydrolyzer is employed in from 0.5 to 15 percent, each based on the total weight of the composition, the balance being water.

5. A process as claimed in Claim 4, wherein the

H$_2$S absorbent concentration is 20 to 50 percent and the COS absorbent/hydrolyzer concentration is 1 to 10 percent.

0227259

6. A process as claimed in any one of the preceding claims, wherein said contact is carried out in a multi-stage contactor for 50 to 120 seconds.

7. A process as claimed in any one of the preceding claims, wherein said contact is conducted at 40 to 90°C.

8. A process as claimed in any one of the preceding claims, which comprises the additional step of:

treating the resulting hydrocarbon stream by contacting the stream with an aqueous caustic solution containing a primary alkanolamine.

9. A process as claimed in Claim 8, wherein the primary alkanolamine is monoethanolamine and is present in from 0.5 to 20 percent by weight and the caustic is present in from 5 to 50 percent by weight, and substantially all of said H$_2$S and CO$_2$ is removed.

10. A process as claimed in any one of the preceding claims, which comprises the further step of:

contacting the so-treated hydrocarbon stream with an aqueous caustic solution in a multi-stage contactor.

11. A process as claimed in Claim 10, wherein each caustic solution is a 5 to 50 percent by weight aqueous solution, the primary alkanolamine is monoethanolamine and is present in from 0.5 to 20 percent by weight.

12. A process as claimed in any one of the preceding claims, wherein the hydrocarbon stream is a liquified petroleum $C_3/C_4$ hydrocarbon stream, which comprises the further steps of:

a) treating the so-treated stream in a $C_3/C_4$ splitter, and, thereafter,

b) treating at least a portion of the lights over-head from said splitter with a caustic solution containing a primary alkanolamine, in a multi-stage contactor.

13. A process as claimed in Claim 12, wherein the primary alkanolamine is monoethanolamine and is present in from 0.5 to 20 percent by weight and the caustic concentration of each of said caustic solutions is from 5 to 50 percent by weight.

14. A process as claimed in any one of Claims 8 to 13, wherein the treatment of the hydrocarbon stream reduces the COS content to less than 1,000 ppb.

0227259

FIG.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DE-A-2 805 640 (EXXON RESEARCH) * claims 1, 2 * | 1-4 | B 01 D 53/14 C 10 G 21/20 C 07 C 7/10 C 07 C 7/11 |
| Y | US-A-4 233 141 (BEAVON et al.) * column 4, lines 60-66; claim 1 * | 1-4 | |
| A | EP-A-0 087 207 (EXXON RESEARCH) * page 2, lines 24-34 * | 1 | |
| A | EP-A-0 077 977 (ASHLAND OIL, INC.) * page 3, paragraph 1; page 5, paragraph 1 * | 1 | |
| A | KIRK-OTHMER "Encyclopedia of Chemical Technology", 3rd edition, vol. 22, 1983, pages 267-282, Wiley-Interscience Publication; * page 269, lines3-17 * | 8,9,12 ,13 | **TECHNICAL FIELDS SEARCHED (Int. Cl.4)** B 01 D 53/00 C 10 G 21/00 C 07 C 7/00 |
| A | DE-A-3 411 532 (BASF) * page 3, lines 1-16 * | 1 | |

The present search report has been drawn up for all claims

| Place of search | Date of completion of the search | Examiner |
|---|---|---|
| BERLIN | 22-01-1987 | PROBERT C.L. |